# EUROPEAN PATENT SPECIFICATION

(11) **EP 1 599 503 B1**
(45) Date of publication and mention of the grant of the patent: **29.12.2010**
(21) Application number: 04717473.5
(22) Date of filing: 04.03.2004
(51) Int. Cl.: C07K 16/28, C07K 16/30, A61K 39/395, A61P 35/00

(54) **ENDOTHELIAL CELL SPECIFIC ANTIBODIES AND USES THEREOF**
ENDOTHELZELL-SPEZIFISCHE ANTIKÖRPER UND IHRE VERWENDUNG
ANTICORPS SPECIFIQUES DES CELLULES ENDOTHELIALES ET LEURS UTILISATIONS

(30) Priority: 04.03.2003 US 451852 P; 04.03.2003 US 451856 P; 06.03.2003 US 452469 P; 18.04.2003 US 463801 P; 24.04.2003 US 465304 P; 16.05.2003 US 471177 P
(43) Date of publication of application: 30.11.2005
(60) Divisional application: 10183529.6
(73) Proprietor: KIRIN BEER KABUSHIKI KAISHA, Tokyo 104-8288 (JP); GENZYME CORPORATION, Framingham, MA 01701 (US)
(72) Inventor: TEICHER, Beverly, Ashland, MA 01721 (US); ROBERTS, Bruce, Southboro, MA 01772 (US); KATAOKA, Shiro, Takasaki-shi, Gunma 370-0044 (JP); TAHARA, Tomoyuki, Takasaki-shi, Gunma, 370-0864 (JP); HONMA, Nakayuki;, Takasaki-shi, Gunma 370-0853; (JP)
(74) Representative: Williams, Richard Andrew Norman
(86) International application number: PCT/US2004/006719
(87) International publication number: WO 2004/078942

(56) References cited:
- WO-A-02/10217
- US-A- 5 342 757
- US-A1- 2003 017 157
- ELEANOR B ET AL: "Cell surface tumor endothelial markers are conserved in mice and humans" CANCER RESEARCH, AMERICAN ASSOCIATION FOR CANCER RESEARCH, BALTIMORE, MD, US, vol. 61, 15 September 2001 (2001-09-15), pages 6649-6655, XP002952961 ISSN: 0008-5472
- RETTIG W J ET AL: "Identification of endosialin, a cell surface glycoprotein of vascular endothelial cells in human cancer." PROCEEDINGS OF THE NATIONAL ACADEMY OF SCIENCES OF THE UNITED STATES OF AMERICA. 15 NOV 1992, vol. 89, no. 22, 15 November 1992 (1992-11-15), pages 10832-10836, XP002294706 ISSN: 0027-8424
- BAGLEY REBECCA ET AL: "Endothelial precursor cells from human bone marrow express tumor endothelial markers (TEMs): Characterization and use in in vitro and in vivo assays." PROCEEDINGS OF THE AMERICAN ASSOCIATION FOR CANCER RESEARCH ANNUAL MEETING, vol. 44, July 2003 (2003-07), page 857, XP008034730 & 94TH ANNUAL MEETING OF THE AMERICAN ASSOCIATION FOR CANCER RESEARCH; WASHINGTON, DC, USA; JULY 11-14, 2003 ISSN: 0197-016X
- BOYER A S ET AL: "TGFbeta2 and TGFbeta3 have separate and sequential activities during epithelial-mesenchymal cell transformation in the embryonic heart." DEVELOPMENTAL BIOLOGY. 15 APR 1999, vol. 208, no. 2, 15 April 1999 (1999-04-15), pages 530-545, XP002294707 ISSN: 0012-1606
- CAO GAOYUAN ET AL: "Involvement of human PECAM-1 in angiogenesis and in vitro endothelial cell migration." AMERICAN JOURNAL OF PHYSIOLOGY. CELL PHYSIOLOGY. MAY 2002, vol. 282, no. 5, May 2002 (2002-05), pages C1181-C1190, XP002294708 ISSN: 0363-6143
- KATO T ET AL: "Diminished corneal angiogenesis in gelatinase A-deficient mice" FEBS LETTERS, ELSEVIER SCIENCE PUBLISHERS, AMSTERDAM, NL, vol. 508, no. 2, 16 November 2001 (2001-11-16), pages 187-190, XP004323227 ISSN: 0014-5793
- HENTGES S ET AL: "Opposing actions of two transforming growth factor-beta isoforms on pituitary lactotropic cell proliferation." ENDOCRINOLOGY. APR 2000, vol. 141, no. 4, April 2000 (2000-04), pages 1528-1535, XP002294709 ISSN: 0013-7227
- WU Y ET AL: "Signaling by a new anti-Thy 1 monoclonal antibody inhibits T cell proliferation and interferes with T-cell-mediated induction of costimulatory molecule B7-2." CELLULAR IMMUNOLOGY. 15 OCT 1995, vol. 165, no. 2, 15 October 1995 (1995-10-15), pages 266-277, XP002294710 ISSN: 0008-8749
- YELIAN F D ET AL: "Recombinant entactin promotes mouse primary trophoblast cell adhesion and migration through the Arg-Gly-Asp (RGD) recognition sequence." THE JOURNAL OF CELL BIOLOGY. MAY 1993, vol. 121, no. 4, May 1993 (1993-05), pages 923-929, XP002294711 ISSN: 0021-9525
- HOLLANDER N: "Antibodies to nonpolymorphic determinants of the Thy-1 molecule inhibit T cell proliferation." JOURNAL OF IMMUNOLOGY (BALTIMORE, MD. : 1950) MAY 1985, vol. 134, no. 5, May 1985 (1985-05), pages 2916-2921, XP002294712 ISSN: 0022-1767
- ALBINI A ET AL: "Tumor cell invasion inhibited by TIMP-2." JOURNAL OF THE NATIONAL CANCER INSTITUTE. 5 JUN 1991, vol. 83, no. 11, 5 June 1991 (1991-06-05), pages 775-779, XP008034940 ISSN: 0027-8874
- ST CROIX B ET AL: "Genes expressed in human tumor endothelium" SCIENCE, AMERICAN ASSOCIATION FOR THE ADVANCEMENT OF SCIENCE,, US, vol. 289, no. 5482, 18 August 2000 (2000-08-18), pages 1197-1292, XP002201336 ISSN: 0036-8075

## Description

### Technical Field

The methods and compositions disclosed herein inhibit proliferation, migration, and/or tubule formation by endothelial cells and thus are useful in treating angiogenesis related disorders and diseases, including cancer. More specifically, the methods relate to the administration of an antibody specific for the human tumor endothelial marker (TEM) 1 in unconjugated form to inhibit tumor growth, and compositions useful in these methods.

### Background of the Invention

Angiogenesis encompasses the generation of new blood vessels in a tissue or organ. Under normal physiological conditions, angiogenesis occurs in very specific situations such as wound healing, fetal development, and the formation of the corpus luteum, endometrium and placenta. The process of angiogenesis is highly regulated through a system of naturally occurring stimulators, *e.g*., angiopoietin-1, IL-8, platelet-derived endothelial cell growth factor (PD-ECGF), and tumor necrosis factor-alpha (TNF-α), and inhibitors, *e.g*., thrombospondin, interferon, and metalloproteinase inhibitors.

Angiogenesis also can occur as a significant factor in a number of disease states. In fact, uncontrolled angiogenesis directly contributes to the pathological damage associated with many diseases. This uncontrolled or excessive angiogenesis occurs when an imbalance in the angiogenic factors and angiogenic inhibitors occurs, *e.g*., when an excessive amount of angiogenic factor is produced. Insufficient angiogenesis also contributes to certain disease states. For example, inadequate blood vessel growth contributes to the pathology associated with coronary artery disease, stroke, and delayed wound healing.

Excessive angiogenesis occurs in diseases such as diabetic retinopathy, age-related macular degeneration, atherosclerosis, and inflammatory conditions such as rheumatoid arthritis and psoriasis. For example, in rheumatoid arthritis, the blood vessels in the synovial lining of the joints undergo inappropriate angiogenesis. In addition to forming new vascular networks, the endothelial cells release factors and reactive oxygen species that lead to pannus growth and cartilage destruction, and thus may actively contribute to, and help maintain, the chronically inflamed state of rheumatoid arthritis. *See*, *e*.*g*., Bodolay, E., et al., J. Cell Mol. Med. 6: 357-76 (2002). Similarly, in osteoarthritis, the activation of the chondrocytes by angiogenic-related factors may contribute to the destruction of the joint. *See, e.g.,* Walsh, D.A., et al., Arthritis Res. 3: 147-53 (2001).

Angiogenesis plays a decisive role in the growth and metastasis of cancer. *See, e.g.,* B.R. Zetter, Ann. Rev. Med. 49: 407-24 (1998), J. Folkman, Sem. Oncol. 29: 15-18 (2002). First, angiogenesis results in the vascularization of a primary tumor, supplying necessary nutrients to the growing tumor cells. Second, the increased vascularization of the tumor provides access to the blood stream, thus enhancing the metastatic potential of the tumor. Finally, after the metastatic tumor cells have left the site of primary tumor growth, angiogenesis must occur to support the growth and expansion of the metastatic cells at the secondary site.

Normal and disease-related angiogenesis seem to proceed in a similar manner. Endothelial cells and pericytes, surrounded by a basement membrane, form capillary blood vessels. Disease or injury induces the production of pro-angiogenic factors. Initially, these factors activate endothelial cells and leukocytes to release enzymes that erode or dissolve the basement membrane of the existing blood vessels. The activated endothelial cells, which line the lumen of blood vessels, then protrude through the basement membrane. Pro-angiogenic stimulants then induce the endothelial cells to migrate through the compromised basement membrane. The migrating cells form a "sprout" off the parent blood vessel, where the endothelial cells begin to proliferate. The endothelial sprouts merge with each other to form capillary loops using adhesion factors, creating new blood vessels. Additional enzymes, *e.g*., matrix metalloproteinases, then digest the tissue at the tip of the sprouting vessel, permitting active tissue remodeling around the new vessel. The newly formed vessels are stabilized by the pericytes (*i.e*., specialized smooth muscle cells). Once stabilized, the new vessels support blood flow.

Numerous compounds have been identified as angiogenesis inhibitors. Exemplary compounds include protamine (Taylor et al., Nature 297:307 (1982)), heparin, steroids (Folkman et al., Science 221:719 (1983) and U.S. Pat. Nos. 5,001,116 and 4,994,443), thalidomide (R.J. D'Amato, et al. Proc. Natl. Acad. Sci. U.S.A. 91: 4082-85 (1994)), TNP-470 (Ingmer, et al., Nature 348: 555-57 (1990)), and carboxyamidotriazole (CAI) (Kohn, et al., Cancer Res. 56: 569-73 (1996). Endogenously produced inhibitors include interferon alpha (IFN-α) (White et al., New England J. Med. 320:1197-1200 (1989), Sidky et al., Cancer Res. 47:5155-61 (1987), angiostatin (O'Reilly, et al., Cell 79: 315-28 (1994)), and endostatin (O'Reilly, et al., Cell 88: 1-20 (1997)).

The limitation of many of these compounds is in their generic nature of anti-angiogenic activity. In other words, most compounds lack the capacity to distinguish between normal angiogenesis and disease-associated angiogenesis. As a result, significant toxicity limits the usefulness of many of the identified anti-angiogenic agents clinically because both normal and disease-related angiogenesis are inhibited. Therefore, an angiogenic inhibitor that selectively targets disease-associated angiogenesis offers significant advantages in decreased toxicity and potentially increased long-term efficacy.
[0008a] Published International Application no. WO 02/10217 discloses the DNA sequences of a number of TEMs as well as the pattern of TEM mRNA expression in normal and tumour endothelial cells. It also discloses that TEM 1 is a cell surface protein. However, while WO 02/10217 additionally refers to TEM 1 antibodies, it does not teach any unconjugated antibody that specifically binds human TEM 1 for use in inhibiting tumor growth. It does not provide any evidence re capability of such an antibody to inhibit endothelial cell proliferation, migration or tube formation *in vitro.*

### Brief Summary of the Invention

In one aspect, the present invention now provides an antibody, or a biologically active fragment thereof, which specifically binds to human Tumor Endothelial Marker (TEM) 1 for use in inhibiting tumor growth.

An effective amount of the antibody, or biologically active antibody fragment, may be administered to inhibit cell migration.

An effective amount of the antibody, or biologically active antibody fragment may be administered to inhibit endothelial tube formation.

The antibody can be a human antibody or a humanized antibody. An intact antibody molecule, a single chain variable region, a Fab fragment, or an F(ab')₂ fragment can be used. The antibody can be a monoclonal antibody, a chimeric antibody, or a polyclonal antibody. The antibody can be produced in a transgenic mouse. In one embodiment, the antibody is specific for an extracellular domain of the antigen.

Also provided herein is an unconjugated antibody, or a biologically active fragment thereof, which specifically binds to human TEM 1 for use in inhibiting tumour growth by inhibiting angiogenesis. The subject treated expresses the TEM of interest. The angiogenesis can be neoangiogenesis. The subject treated may be a subject having a disease including, but are not limited to neoplasma of the central nervous system: glioblastomamultiforme, astrocytoma, oligodendroglial tumors, ependymal and choroids plexus tumors, pineal tumors, neuronal tumors, medulloblastoma, schwannoma, meningioma, meningeal sarcoma: neoplasma of the eye: basal cell carcinoma, squamous cell carcinoma, melanoma, rhabdomyosarcoma, retinoblastoma; neoplasma of the enbdocrine glands: pituitary neoplasms, neoplasms of the thyroid, neoplasms of the adrenal cortex, neoplasms of the neuroendocrine system, neoplasms of the gastroenteropancreatic endocrine system, neoplasms of the gonads; neoplasms of the head and neck: head and neck cancer, oral cavity, pharynx, larynx, odontogenic tumors: neoplasms of the thorax: large cell lung carcinoma, small cell lung carcinoma, non-small cell lung carcinoma, neoplasms of the thorax, malignant mesothelioma, thymomas, primary germ cell tumors of the thorax; neoplasms of the alimentary canal: neoplasms of the esophagus, neoplasms of the stomach, neoplasms of the liver, neoplasms of the gallbladder, neoplasms of the exocrine pancreas, neoplasms of the small intestine, vermiform appendix and peritoneum, adenocarcinoma of the colon and rectum, neoplasms of the anus;
neoplasms of the genitourinary tract: renal cell carcinoma, neoplasms of the renal pelvis and ureter, neoplasms of the bladder, neoplasms of the urethra, neoplasms of the prostate, neoplasms of the penis, neoplasms of the testis; neoplasms of the female reproductive organs: neoplasms of the vulva and vagina, neoplasms of the cervix, adenocarcinoma of the uterine corpus, ovarian cancer, gynecologic sarcomas; neoplasms of the breast; neoplasms of the skin: basal cell carcinoma, squamous carcinoma, dermatofibrosarcoma, Merkel cell tumor; malignant melanoma; neoplasms of the bone and soft tissue: osteogenic sarcoma, malignant fibrous histiocytoma, chrondrosarcoma, Ewing's sarcoma, primitive neuroectodermal tumor, angiosarcoma; neoplasms of the hematipoeitic system: myelodysplastic syndromes, acute myeloid leukemia, chronic myeloid leukemia, acute lymphocytic leukemia, HTLV-1, and T-cell leukemia/lymphoma, chronic lymphocytic leukemia, hairy cell leukemia, Hodgkin's disease, non-Hodgkin's lymphomas, mast cell leukemia; neoplasms of children: acute lymphoblastic leukemia, acute myelocytic leukemias, neuroblastoma, bone tumors, rhabdomyosarcoma, lymphomas, renal and liver tumors. Other diseases include polycystic kidney disease; diabetic retinopathy; rheumatoid arthritis; psoriasis; osteoarthritis; acoustic neuroma, neurofibroma, trachoma and pyogenic granulomas; macular degeneration; retinopathy of prematurity; corneal graft rejection; neovascular glaucoma and retrolental fibroplasia. Other diseases associated with angiogenesis include, but are not limited to, epidemnic keratoconjunctivitis, Vitamin A deficiency, atopic keratitis, superior limbic keratitis, pterygium keratitis sicca, sjogrens, phylectenulosis, syphilis, Mycobacteria infections, lipid degeneration, chemical bums, bacterial ulcers, fungal ulcers, Herpes simplex infections, Herpes zoster infections, protozoan infections, Kaposi sarcoma, Mooren ulcer, Terrien's marginal degeneration, marginal keratolysis, systemic lupus, polyarteritis, trauma, Wegeners sarcoidosis, Scleritis, Steven's Johnson disease, periphigoid radial keratotomy, corneal graph rejection, and chronic inflammatory diseases.

As herein before indicated, however, in its broadest aspect the invention as claimed provides an unconjugated antibody, or a biologically active fragment thereof, which specifically binds to human TEM 1 for use in inhibiting tumor growth where the subject treated expresses the TEM of interest. The subject treated may be a subject having an adenocarcinoma, leukemia, lymphoma, melanoma, sarcoma, or tetratocarcinoma. The tumor can be a cancer of the adrenal gland, bladder, bone, bone marrow, brain, breast, cervix, gall bladder, ganglia, gastrointestinal tract, heart, kidney, liver, lung, muscle, ovary, pancreas, parathyroid, penis, prostate, salivary glands, skin, spleen, testis, thymus, thyroid, and uterus. Such tumors include, but are not limited to: neoplasma of the central nervous system: glioblastomamultiforme, astrocytoma, oligodendroglial tumors, ependymal and choroids plexus tumors, pineal tumors, neuronal tumors, medulloblastoma, schwannoma, meningioma, meningeal sarcoma: neoplasma of the eye: basal cell carcinoma, squamous cell carcinoma, melanoma, rhabdomyosarcoma, retinoblastoma; neoplasma of the endocrine glands: pituitary neoplasms, neoplasms of the thyroid, neoplasms of the adrenal cortex, neoplasms of the neuroendocrine system, neoplasms of the gastroenteropancreatic endocrine system, neoplasms of the gonads; neoplasms of the head and neck: head and neck cancer, oral cavity, pharynx, larynx, odontogenic tumors: neoplasms of the thorax: large cell lung carcinoma, small cell lung carcinoma, non-small cell lung carcinoma, neoplasms of the thorax, malignant mesothelioma, thymomas, primary germ cell tumors of the thorax; neoplasms of the alimentary canal: neoplasms of the esophagus, neoplasms of the stomach, neoplasms of the liver, neoplasms of the gallbladder, neoplasms of the exocrine pancreas, neoplasms of the small intestine, vermiform appendix and peritoneum, adenocarcinoma of the colon and rectum, neoplasms of the anus; neoplasms of the genitourinary tract: renal cell carcinoma, neoplasms of the renal pelvis and ureter, neoplasms of the bladder, neoplasms of the urethra, neoplasms of the prostate, neoplasms of the penis, neoplasms of the testis; neoplasms of the female reproductive organs: neoplasms of the vulva and vagina, neoplasms of the cervix, adenocarcinoma of the uterine corpus, ovarian cancer, gynecologic sarcomas; neoplasms of the breast; neoplasms of the skin: basal cell carcinoma, squamous carcinoma, dermatofibrosarcoma, Merkel cell tumor; malignant melanoma; neoplasms of the bone and soft tissue: osteogenic sarcoma, malignant fibrous histiocytoma, chrondrosarcoma, Ewing's sarcoma, primitive neuroectodermal tumor, angiosarcoma; neoplasms of the hematipoeitic system: myelodysplastic syndromes, acute myeloid leukemia, chronic myeloid leukemia, acute lymphocytic leukemia, HTLV-1, and T-cell leukemia/lymphoma, chronic lymphocytic leukemia, hairy cell leukemia, Hodgkin's disease, non-Hodgkin's lymphomas, mast cell leukemia; neoplasms of children: acute lymphoblastic leukemia, acute myelocytic leukemias, neuroblastoma, bone tumors, rhabdomyosarcoma, lymphomas, renal and liver tumors.

Further provided herein as discussed further below are compositions of an antibody useful in accordance with the invention.

Disclosed herein is a method for identifying an angiogenesis inhibitor molecule that modulates TEM 1, which comprises contacting a TEM 1-transgenic mouse bearing a tumor with a test molecule; and detecting the inhibition of the tumor growth, whereby the test molecule is identified as an angiogenesis inhibitor molecule that modulates TEM 1 when the tumor growth in the mouse contacted with the test molecule is reduced relative to the tumor growth in the mouse not contacted by the test molecule.

Disclosed herein, but not part of the claimed invention, is also a method for identifying an angiogenesis inhibitor molecule that modulates TEM 9, which comprises contacting a TEM 9-transgenic mouse bearing a tumor with a test molecule; and detecting the inhibition of the tumor growth, whereby the test molecule is identified as an angiogenesis inhibitor molecule that modulates TEM 9 when the tumor growth in the mouse contacted with the test molecule is reduced relative to the tumor growth in the mouse not contacted by the test molecule.

Disclosed herein, but not part of the claimed invention, is additionally a method for identifying an angiogenesis inhibitor molecule that modulates TEM 17, which comprises contacting a TEM 17-transgenic mouse bearing a tumor with a test molecule; and detecting the inhibition of the tumor growth, whereby the test molecule is identified as an angiogenesis inhibitor molecule that modulates TEM 17 when the tumor growth in the mouse contacted with the test molecule is reduced relative to the tumor growth in the mouse not contacted by the test molecule.

Further provided herein is a monoclonal antibody, or a biologically active fragment thereof, which specifically binds human TEM 1 for administration in accordance with the invention, wherein the antibody is produced by a hybridoma. In one embodiment the hybridoma is TEM1-70. In another embodiment, the hybridoma is TEM1-7. In yet another embodiment, the hybridoma is TEM1-38. Also provided herein is the monoclonal antibody, or a biologically active fragment thereof, wherein the antibody comprises the same sequence of amino acids of the variable region of the antibody specific for TEM 1. In one embodiment, the antibody is an IgG antibody. In a specific embodiment, the IgG antibody is selected from the members of an IgG subclass consisting of IgG₁, IgG₂, IgG₃, and IgG₄. At least one amino acid of the heavy chain of the antibody can be altered with a deletion, addition, or substitution with an amino acid different from the original amino acid. In one embodiment, the antibody inhibits angiogenesis that promotes or causes cancer. As hereinbefore indicated, the TEM1-specific antibody inhibits tumor growth such as a growth of a tumor which is an adenocarinoma, leukemia, lymphoma, melanoma, sarcoma, or tetracarcinoma. The tumor can be a cancer of the adrenal gland, bladder, bone, bone marrow, brain, breast, cervix, gall bladder, ganglia, gastrointestinal tract, heart, kidney, liver, lung, muscle, ovary, pancreas, parathyroid, penis, prostate, salivary glands, skin, spleen, testis, thymus, thyroid, or uterus.

Disclosed herein is a pharmaceutical composition comprising an amount of the monoclonal antibody, or a biologically active fragment thereof, of the monoclonal antibody specific for TEM1 and a suitable excipient. The antibody may be produced by TEM1-70. The antibody may be produced by TEM1-38 or TEM1-7.

Provision of the hybridomas TEM1-70, TEM1-7, and TEM1-38 is further discussed in the examples.

### Detailed Description of the Invention

The antibodies and uses thereof disclosed herein relate to the growth and function of the endothelial cell *in vitro* and *in vivo.* Specifically, as indicated above, antibodies of interest are specific for the surface molecule on endothelial cells known as TEM 1, a molecule that is preferentially expressed on endothelial cells found within tumors. Therefore, these antibodies preferentially target disease-related angiogenesis. Antibody molecules that target TEMs can be useful as modulators of endothelial cell activity (*e.g*., as a therapeutic agent) through at least 4 different mechanisms. First, an antibody can act directly on its target cell by modulating or inhibiting key macromolecular signal transduction pathways. For example, an antibody can bind a critical ligand or its receptor and inhibit a necessary positive stimulus or, alternatively, induce a negative signal (*e.g*., one eliciting apoptosis). Second, an antibody can act indirectly on its target cell by simply binding its target and deliver a bioactive agent as its conjugate, *e.g*., a radionuclide or potent toxin with therapeutic benefit. Third, an antibody can engage other components of the immune system to act on the target cell. For example, the antibody can be used to trigger antibody-dependent cellular cytotoxicity (ADCC) or complement-dependent cytolysis (CDC). Fourth, the antibody can have intrinsic cytotoxicity.

Targeting tumor vasculature endothelium has several unique advantages. First, there is a minimal barrier between the luminal surface of vascular endothelium and intravenously administered agents, enhancing the efficiency of delivery to the targeted cells. Second, localized damage of a few endothelial cells can lead to vascular occlusion as a result of reactive swelling of the endothelium and compromise the surrounding endothelial cells, allowing the localized damage to achieve a more global effect. Third, endothelial cells may not develop resistance to the antibody, permitting repeated administration of the antibody without any decrease in its efficacy.

As used herein, the term "tumor endothelial marker (TEM)" refers to a molecule preferentially expressed on tumor endothelial cells. The expression of TEMs is absent or significantly lower on normal (non-tumor) vasculature. *See, e.g.,* St. Croix, et al., Science 289: 1197-1202 (2000), U.S. Serial No. 09/918715 (Publication No. 20030017157). TEM encompasses any type of molecule expressed on the surface of an endothelial cell for at least a portion of its half-life. Such molecules include, but are not limited to TEM 1, TEM 2, TEM 3, TEM 4, TEM 5, TEM 6, TEM 7, TEM 8, TEM 9, TEM 10, TEM 11, TEM 12, TEM 13, TEM 14, TEM 15, TEM 16, TEM 17, TEM 18, TEM 19, TEM 20, TEM 21, TEM 22, TEM 23, TEM 24, TEM 25, TEM 26, TEM 27, TEM 28, TEM 29, TEM 30, TEM 31, TEM 32, TEM 33, TEM 34, TEM 35, TEM 36, TEM 37, TEM 38, TEM 39, TEM 40, TEM 41, TEM 42, TEM 43, TEM 44, TEM 45, and TEM 46.

For the purpose of the invention, the target molecule is TEM 1. Endosialin is a 165 kDa glycoprotein. Rettig, et al., Proc. Nat'l Acad. Sci. U.S.A. 89: 10832-36 (1992), Christian, et al., J. Biol. Chem. 276: 7408-14 (2001). The sequence of TEM 1 was identified as that of the endosialin protein by St. Croix and colleagues in Science 289: 1197-1202 (2001). TEM 1 is a C-type lectin-like, type I membrane protein with a signal leader peptide, five globular extracellular domains, followed by a mucin-like region, a transmembrane segment and a short cytoplasmic tail. The N-terminal shows homology to thrombomdulin, a receptor involved in regulating blood coagulation and to complement receptor C1qRp. Webster, et al., J. Leuk. Biol. 67: 109-16 (2000). Murine and human TEM 1 share 77.5% amino acid identity with 100% identity in the transmembrane region. Opavsky, et al., J. Biol. Chem. 276: 38795-807 (2001). TEM 1 has a signal sequence at amino acids 1-17 and its transmembrane domain at amino acids 686-708. Its extracellular domain is at residues 1-685. TEM 1 expression varies with cell density (or cell cycle). Opavsky *et al., supra* (2001). TEM 1 is maximally expressed in confluent (G₀) cells, the most relevant phase of the cell cycle *in vivo.* The DNA sequence of TEM 1 is disclosed as SEQ ID NO. 196 in U.S. Serial No. 09/918715 (Publication No. 20030017157).

Another TEM is TEM 17. TEM 17 is a Type I membrane protein with a signal sequence at residues 1-18, a N-terminal region similar to the G1 domain of nidogen, a 100 amino acid region with homology to plexins, a transmembrane domain at residues 427-445, and a short cytoplasmic tail. Its extracellular region comprises residues 1-426. The plexin family mediates cell guidance cues, suggesting that TEM 17 may function in this capacity. Murine TEM 17 has 81% sequence identity with the human TEM 17. The DNA sequence of TEM 17 is disclosed as SEQ ID NO. 230 in U.S. Serial No. 09/918715 (Publication No. 20030017157).

A further TEM is TEM 9. TEM 9 is a secretin family seven-span transmembrane G-protein coupled receptor. This G- protein coupled receptor homologue has both a signal sequence at residues 1-26 and 7 transmembrane domains. The N-terminal extracellular domain consists of a leucine-rich repeat (LRR) region, followed by an immunoglobulin domain, a peptide hormone receptor domain, and a GPCR proteolytic site domain. Its extracellular region resides in amino acids 1-769, and its transmembrane domains are at residues 817-829 (TM2 and TM3), residues 899-929 (TM4 and TM5), and residues 1034-1040 (TM6 and TM7). The transmembrane region is homologous to the transmembrane region of other secretin-family serpentine receptor. The beginning of the cytoplasmic region contains two vicinal cysteines typical for the GPCR-palmitoylation. Thus, TEM 9 is a G-protein coupled receptor with extracellular domains characteristic of cell adhesion proteins. The mouse ortholog has a predicted signal peptide at residues 1-29 and has 87% homology to the human TEM 9. The DNA sequence of TEM 9 is disclosed as SEQ ID NO. 212 in U.S. Serial No. 09/918715 (Publication No. 20030017157).

Any antibody specific for human TEM 1 can be used in accordance with the invention. As used herein, the term "antibody" refers to a molecule comprising at least one variable region from a light chain immunoglobulin molecule and at least one variable region from a heavy chain molecule that in combination form a specific binding site for the target antigen. An antibody can be useful in the form of a Fab fragment, F(ab')₂ fragment, a single chain variable region, or an intact antibody molecule. Fragments of intact molecules can be generated using methods well known in the art and include enzymatic digestion and recombinant means. The fragments useful in the present methods are biologically active fragments. As used herein, the term "biologically active" refers to an antibody or antibody fragment that is capable of binding the desired antigenic epitope and directly exerting a biologic effect. Direct effects include, but are not limited to the inhibition of a growth signal, the inhibition of an anti-apoptotic signal, the elicitation of an apoptotic or necrotic signal, initiating the ADCC cascade, and initiating the CDC cascade. As used herein, the term "specific" refers to the selective binding of the antibody to the target antigen epitope. Antibodies can be tested for specificity of binding by comparing binding to appropriate antigen to binding to irrelevant antigen or antigen mixture under a given set of conditions. If the antibody binds to the appropriate antigen at least 2, 5, 7, and preferably 10 times more than to irrelevant antigen or antigen mixture then it is considered to be specific. In one embodiment, a specific antibody is one that binds the human TEM antigen of interest, but does not bind a non-human TEM antigen, *e*.*g*., murine TEM antigen. In another embodiment, a specific antibody is one that binds the human TEM antigen but does not bind a non-human TEM antigen with 70%, 75%, 80%, 85%, 90%, 95%, 97% or greater amino acid homology with the human TEM antigen. In one embodiment, the antibody is an IgG antibody. For example, the antibody is a IgG₁, IgG₂, IgG3, or IgG4 antibody.

In one embodiment, the antibody is clone 7, which is specific for TEM 1, and is produced by the hybridoma TEM1-7. In another embodiment, the antibody is clone 70, which is specific for TEM 1, and is produced by hybridoma TEM1-70. In yet another embodiment, the antibody is clone 38, which is specific for TEM 1, and is produced by hybridoma TEM1-38. Hybridomas producing clone 7 (TEM1-7), clone 38 (TEM1-38), and clone 70 (TEM1-70) were internationally deposited on May 15, 2003, with the National Institute of Advanced Industrial Science and Technology, International Patent Organism Depositary (AIST Tsukuba Central 6, 1-1-1 Higashi, Tsukuba, Ibaraki, 305-8566, Japan) under the provisions of the Budapest Treaty, having the accession numbers FERM BP-8380 (TEM1-7), FERM BP-8381 (TEM1-38), and FERM BP-8382 (TEM1-70).

The antibodies useful in accordance with the invention can be generated in cell culture, in phage, or in various animals, including but not limited to cows, rabbits, goats, mice, rats, hamsters, guinea pigs, sheep, dogs, cats, monkeys, chimpanzees, apes. Therefore, the antibody useful in the present methods is a mammalian antibody. Phage techniques can be used to isolate an initial antibody or to generate variants with altered specificity or avidity characteristics. Such techniques are routine and well known in the art. In one embodiment, the antibody is produced by recombinant means known in the art. For example, a recombinant antibody can be produced by transfecting a host cell with a vector comprising a DNA sequence encoding the antibody. One or more vectors can be used to transfect the DNA sequence expressing at least one VL and one VH region in the host cell. Exemplary descriptions of recombinant means of antibody generation and production include Delves, ANTIBODY PRODUCTION: ESSENTIAL TECHNIQUES (Wiley, 1997); Shephard, et al., MONOCLONAL ANTIBODIES (Oxford University Press, 2000); and Goding, MONOCLONAL ANTIBODIES: PRINCIPLES AND PRACTICE (Academic Press, 1993). The antibody useful in the present methods can be modified by recombinant means to increase greater efficacy of the antibody in mediating the desired function. It is also contemplated that antibodies can be modified by substitutions using recombinant means. Typically, the substitutions will be conservative substitutions. For example, at least one amino acid in the constant region of the antibody can be replaced with a different residue. See, e.g., U.S. Patent No. 5,624,821, U.S. Patent No. 6,194,551, Application No. WO 9958572; and Angal, et al., Mol. Immunol. 30 : 105-08 (1993). The modification in amino acids includes deletions, additions, substitutions of amino acids. In some cases, such changes are made to reduce undesired activities, *e.g*., complement-dependent cytotoxicity.

The antibody can be a humanized antibody. As used herein, the term "humanized antibody" refers to an antibody where the amino acid sequence in the non-antigen binding regions are altered so that the antibody more closely resembles a human antibody while still retaining it original antigen specificity. Typically, the variable regions are of one species, *e.g*., mouse, and the constant regions are human in origin. The antibody can be a chimeric antibody. As used herein, the term "chimeric antibody" refers to an antibody where the amino acid sequences are altered so that the antibody contains sequences from more than one mammal while still retaining it original antigen specificity. As used herein, the term "single-chain variable fragment (ScFv)" refers to a genetically engineered antibody that consists of the variable heavy chain (V_{H}) and light chain (V_{L}) of an immunoglobulin joined together by a flexible peptide linker.

Any form of the antigen can be used to generate the antibody that is sufficient to generate a biologically active antibody. Thus, the eliciting antigen may be a single epitope, multiple epitopes, or the entire protein alone or in combination with one or more immunogenicity enhancing agents known in the art. The eliciting antigen may be an isolated full-length protein, a cell surface protein (*e.g*., immunizing with cells transfected with at least a portion of the antigen), or a soluble protein (*e.g*., immunizing with only the extracellular domain portion of the protein). The antigen may be produced in a genetically modified cell. The DNA encoding the antigen may genomic or non-genomic (*e.g*., cDNA) and encodes at least a portion of the extracellular domain. As used herein, the term "portion" refers to the minimal number of amino acids or nucleic acids, as appropriate, to constitute an immunogenic epitope of the antigen of interest. Any genetic vectors suitable for transformation of the cells of interest may be employed, including but not limited to adenoviral vectors, plasmids, and non-viral vectors, such as cationic lipids. In one embodiment, the antibody employed specifically binds at least a portion of the extracellular domain of the TEM of interest.

The antibody can be monoclonal or polyclonal. As used herein, the term "monoclonal antibody" refers to a singular antibody produced by a single B cell. As used herein, the term "polyclonal antibody" refers to a mixture of monoclonal antibodies with the same antigen specificity, but being produced by more than one B cell clone. In one embodiment, the polyclonal antibody contains monoclonal antibodies with different epitope specificities, affinities, or avidities within a single antigen that contains multiple antigenic epitopes.

In one embodiment, the antibody provided herein is a human antibody. As used herein, the term "human antibody" refers to an antibody in which essentially the entire sequences of the light chain and heavy chain sequences, including the complementary determining regions (CDRs), are from human genes. In one embodiment, human monoclonal antibodies are prepared by the trioma technique, the human B-cell technique (*see, e.g.,* Kozbor, et al., Immunol. Today 4; 72 (1983), EBV transformation technique (*see, e.g.,* Cole et al. MONOCLONAL ANTIBODIES AND CANCER THERAPY 77-96 (1985)), or using phage display (*see, e.g.,* Marks et al., J. Mol. Biol. 222:581 (1991)). In a specific embodiment, the human antibody is generated in a transgenic mouse. Techniques for making such partially to fully human antibodies are known in the art and any such techniques can be used. According to one particularly preferred embodiment, fully human antibody sequences are made in a transgenic mouse engineered to express human heavy and light chain antibody genes. An exemplary description of preparing transgenic mice that produce human antibodies found in PCT Publication No. WO 02/43478. B cells from transgenic mice that produce the desired antibody can then be fused to make hybridoma cell lines for continuous production of the antibody. *See, e.g.,* U.S. Patent Nos. 5,569,825; 5,625,126; 5,633,425; 5,661,016; and 5,545,806; and Jakobovits, Adv. Drug Del. Rev. 31: 33-42 (1998); Green, et al., J. Exp. Med. 188: 483-495 (1998).

Bispecific antibodies are also useful in accordance with the invention. As used herein, the term "bispecific antibody" refers to an antibody, typically a monoclonal antibody, having binding specificities for at least two different antigenic epitopes. In one embodiment, the epitopes are from the same antigen. In another embodiment, the epitopes are from two different antigens. Methods for making bispecific antibodies are known in the art. For example, bispecific antibodies can be produced recombinantly using the co-expression of two immunoglobulin heavy chain/light chain pairs. *See, e.g.,* Milstein et al., Nature 305: 537-39 (1983). Alternatively, bispecific antibodies can be prepared using chemical linkage. *See, e.g.,* Brennan, et al., Science 229: 81 (1985). Bispecific antibodies include bispecific antibody fragments. *See, e.g.,* Hollinger, et al., Proc. Natl. Acad. Sci. U.S.A. 90: 6444-48 (1993), Gruber, et al., J. Immunol. 152: 5368 (1994).

Heteroconjugate antibodies are useful in accordance with the invention. As used herein, the term "heteroconjugate antibody" refers to two covalently joined antibodies. Such antibodies can be prepared using known methods in synthetic protein chemistry, including using crosslinking agents. *See, e.g.,* U.S. Patent No. 4,676,980.

It is further emphasised that the antibodies provided for use in accordance with the claimed invention are unconjugated.

The antibodies provided herein are also useful in methods to identify the presence or expression of TEM 1. Such a method for defecting the TEM polypolypeptide comprises contacting a sample with a TEM-specific antibody for a period sufficient to form a complex, and detecting the complex, so that if a complex is detected, the TEM is present in the sample. Examples of such assays include radioimmunoassays, ELISAs, immunochemistry, immunoprecipitation, and other well known immunoassays and are found in, *e*.*g*., USING ANTIBODIES: A LABORATORY MANUAL (Harlow, et al., ed.s 1999). The samples include, but are not limited to cells, protein or membrane extracts of cells, biological fluids such as sputum, blood, serum, plasma, or urine, or biological samples such as formalin-fixed or frozen tissue sections. Methods of preparing such samples for analysis are known in the art. The sample analyzed using the antibodies disclosed herein will vary based on the assay format, nature of the detection method, and the tissues, cells, or extracts of cells to be assayed. Such preparations and variations are well known in the art. Any suitable detection system can be used. Such a detection method can be used to diagnosis, stage, or monitor disease progression or monitor responsiveness to therapeutic intervention.

Specifically now disclosed is a compartment kit comprising one or more containers, wherein a first container comprises one or more antibodies specific to the TEM of interest and one or more other containers comprising one or more of the following: wash reagents, reagents capable of detecting presence of a bound antibody. The containers can be glass, plastic, or strips of plastic or paper. Types of detection agents include labeled secondary antibodies, other labeled secondary binding agents, or in the alternative, if the primary antibody is labeled, the enzymatic, or antibody binding reagents that are capable of reacting with the labeled antibody.

Where an effective amount of a human antibody, or a biologically active fragment thereof, which specifically binds to TEM 1 is shown to inhibit cell proliferation, any suitable method for determining cellular proliferation may be used. In one embodiment, the method of inhibiting proliferation in a cell comprises the step of contacting endothelial cells with a TEM 1 specific antibody or biologically active fragment thereof as described above, for a certain duration and then determining proliferation relative to a cell not treated with the antibody.

An antibody is inhibitory for proliferation if it inhibits the proliferation of cells relative to the proliferation of cells in the absence of the antibody or in the presence of a non-binding antibody. Proliferation may be quantified using any suitable methods. Typically, the proliferation is determined by assessing the incorporation of radioactive-labeled nucleotides into DNA (*e.g*., ³H-thymidine). In one embodiment, proliferation is determined by ATP luminescence. In a specific embodiment, proliferation is determined using the CellTiter-Glo™ Luminescent Cell Viability Assay from Promega. The proliferation of a cell also may be inhibited by the administration of the antibody to a tissue or to a subject as described below.

Antibodies that block the function of TEMs associated with enhanced proliferation of tumor endothelial cells are most beneficial to subjects whose malignant disease generates low levels or no circulating endothelial precursor cells, thus indicating that the tumor vasculature of their disease originates, in larger part, from proliferation of endothelial cells on co-opted existing vessels. Blocking the function of TEMS associated with proliferation of tumor endothelial cells also benefits subjects with a relatively lower tumor burden because inhibition of tumor endothelial cell proliferation results in a kinetically slow response to therapy.

Where an effective amount of a human antibody, or a biologically active fragment thereof, which specifically binds to TEM 1 is shown to inhibit cell migration, any suitable method for assessing cell migration may be used. In one embodiment, the method of inhibiting the migration of a cell comprises the steps of contacting endothelial cells with a TEM 1 specific antibody or biologically active fragment thereof as described above in one chamber without the chemoattractant, incubating the endothelial cells and antibody within a chamber separated by a porous membrane from a second chamber with a chemoattractant, determining the number of cells entering the chamber with the chemoattractant in the presence or absence of the antibody, where the antibody is inhibitory to migration when the number of cells migrating in the absence of the antibody is greater than the number migrating in the presence of the antibody.

An antibody is inhibitory for migration if the number of cells migrating in the presence of the antibody is less relative to the migration of cells in the absence of the antibody or in the presence of a non-binding antibody or other irrelevant antibody. Migration may be quantified using any suitable method. Typically, migration is determined by assessing the number of cells in the chamber with the chemoattractant versus those remaining in the original chamber. In one embodiment, the cells are pre-labeled with PKH67 green dye, according to manufacturer's instructions. In another embodiment, the cells are labeled with Calcein AM. Following incubation, the cells are enumerated using a fluorescent inverted phase microscope. The migration of a cell may also be inhibited by the administration of the antibody to a tissue or to a subject as described below.

Antibodies that block the function of TEMs associated with enhanced migration of tumor endothelial cells are most beneficial to subjects whose malignant disease is relatively slow growing or indolent and/or who have known metastatic disease because blood vessel formation in the disease sites of these subjects most likely draws on endothelial precursor cells from the bone marrow as well as endothelial cells from the normal tissue sites of disease, thus requiring migration of endothelial cells as a major function.

Where an effective amount of a human antibody, or a biologically active fragment thereof, which specifically binds to TEM 1 is shown to inhibit endothelial tube formation, any suitable method for assessing tubule formation may be used. In one embodiment, the method of inhibiting the endothelial tubule formation of a cell comprises the steps of contacting endothelial cells with a TEM 1 specific antibody or biologically active fragment thereof as described above in the presence of a suitable matrix, incubating the endothelial cells and antibody within the matrix, assessing the tubule formation, where the antibody is inhibitory to tubule formation when the number of tubules formed or the character of the tubules in the absence of the antibody is greater or significantly altered relative to the number of tubules or the character of the tubules in the presence of the antibody.

An antibody is inhibitory for tubule formation if the number of tubules formed in the presence of the antibody is less relative to the number of tubules formed in the absence of the antibody or in the presence of a non-binding antibody. An antibody is inhibitory for tubule formation if the character of tubules formed in the presence of the antibody is altered relative to the character of the tubules formed in the absence of the antibody or in the presence of a non-binding antibody. As used herein, the term "character of the tubule" refers to the robustness and duration of the tubule networks formed in the matrix. Tubule formation may be quantified using any suitable methods. Typically, tubule formation is assessed by microscopy. In one embodiment, the cells are pre-labeled with PKH67 green dye, according to manufacturer's instructions. In another embodiment, the cells are labeled with Calcein AM. Following incubation with or without antibody in the matrix, the tubules are examined using a fluorescent inverted phase microscope. The tubule formation by a cell also may be inhibited by the administration of the antibody to a tissue or to a subject as described below.

Antibodies that block the function of TEMs associated with enhanced tube formation of tumor endothelial cells are most beneficial to subjects whose malignant disease generates high circulating levels of endothelial precursor cells, thus indicating that the tumor vasculature of their disease originates, in large part, from development of vessel tubes established by circulating endothelial precursor cells. Blocking the function of TEMs associated with enhanced tube formation of tumor endothelial cells also benefits subjects with a relatively rapidly growing tumor where disruption of or inhibition of vessel tube formation blocks continued expansion of the tumor mass.

In applying the invention to inhibit angiogenesis, proliferation, migration, and/or endothelial tubule formation, any cell that expresses the human TEM of interest, or can be induced to express the human TEM of interest, may be targeted. A suitable antibody will target a cell line that endogenously expresses the TEM of interest. Examples of cells induced to express the TEM of interest are AC133+/CD34+ human bone marrow cells cultured in the presence of basic FGF (bFGF), VEGF, and heparin to generate endothelial precursor cells (EPCs) as described in Example 1. Cells may be transfected or transduced with the TEM of interest, for example, the human umbilical vein endothelial cell (HUVEC) or the human microvascular endothelial cell may be transduced with an adenoviral vector encoding the TEM of interest. Alternatively, COS or 293 cells may be transfected with a plasmid encoding the TEM of interest. The TEM-expressing cells may be contacted with growth factors or media conditioned by other cells. For example, colon carcinoma conditioned media can be prepared using confluent cultures of human HCT116 colon carcinoma cells or human HT29 colon carcinoma cells grown in serum free media for 3 days. Factors useful in supplementing media include VEGF and bFGF. In one embodiment, the cell is derived from or in a human or veterinary subject.

The TEM-expressing cell can be contacted with the antibody in any suitable manner for any suitable length of time. The cells can be contacted with the antibody more than once during incubation or treatment. Typically, the dose required is in the range of about 1 µg/ml to 1000 µg/ml, more typically in the range of 100 µg/ml to 800 µg/ml. The exact dose can be readily determined from *in vitro* cultures of the cells and exposure of the cell to varying dosages of the antibody. Typically, the length of time the cell is contacted with the antibody is 1 hour to 3 days, more typically for 24 hours. Any suitable matrix may be used. In one embodiment, the matrix is reconstituted basement membrane Matrigel™ matrix (BD Sciences).

As herein before indicated, the invention is of particular interest to inhibit angiogenesis, comprising administering to a subject in need thereof, an effective amount of a human antibody, or a biologically active fragment thereof, which specifically binds to TEM 1, whereby the antibody inhibits angiogenesis. As used herein, the term "angiogenesis" refers to the development of blood vessels. The blood vessels can be established or new blood vessels. As used herein, the term "neoangiogenesis" refers to the development of new blood vessels.

The invention can be applied to inhibit tumor growth, comprising administering to a subject in need thereof, an effective amount of a human antibody, or a biologically active fragment thereof, which specifically binds to TEM 1, whereby the antibody inhibits the tumor growth.

Subjects thus treated will express the TEM antigen of interest. A screening or diagnostic analysis of patient samples can be performed prior to the initiation of treatment using TEM-specific therapy. Such diagnosis analysis can be performed using any sample, including but not limited to cells, protein or membrane extracts of cells, biological fluids such as sputum, blood, serum, plasma, or urine, or biological samples such as formalin-fixed or frozen tissue sections employing the antibodies of the present invention. Any suitable method for detection and analysis of TEM expression can be employed.

The subject treated in accordance with the invention may be a human with an angiogenesis associated cancer.

As used herein, "inhibit" or "treat" or "treatment" includes a postponement of development of the symptoms associated with uncontrolled angiogenesis, tumor growth and/or a reduction in the severity of such symptoms that will or are expected to develop. The terms further include ameliorating existing uncontrolled or unwanted angiogenesis-related or tumor growth-related symptoms, preventing additional symptoms, and ameliorating or preventing the underlying metabolic causes of symptoms. Thus, the terms denote that a beneficial result has been conferred on a vertebrate subject with an angiogenesis-associated disease or symptom, particularly cancer, or with the potential to develop such a disease or symptom.

As used herein, the term "therapeutically effective amount" or "effective amount" refers to an amount of an anti-TEM antibody that when administered alone or in combination with an additional therapeutic agent to a cell, tissue, or subject is effective to prevent or ameliorate the angiogenic- and/or tumor-associated disease condition or the progression of the disease. A therapeutically effective dose further refers to that amount of the compound sufficient to result in amelioration of symptoms, *e.g*., treatment, healing, prevention or amelioration of the relevant medical condition, or an increase in rate of treatment, healing, prevention or amelioration of such conditions. When applied to an individual active ingredient administered alone, a therapeutically effective dose refers to that ingredient alone. When applied to a combination, a therapeutically effective dose refers to combined amounts of the active ingredients that result in the therapeutic effect, whether administered in combination, serially or simultaneously.

An antibody useful in accordance with the invention (from whatever source derived, including without limitation from recombinant and non-recombinant sources) may be administered to a subject in need, by itself, or in pharmaceutical compositions where it is mixed with suitable carriers or excipient(s) at doses to treat or ameliorate a variety of disorders. Such a composition may also contain (in addition to protein and a carrier) diluents, fillers, salts, buffers, stabilizers, solubilizers, and other materials well known in the art. The term "pharmaceutically acceptable" means a non-toxic material that does not interfere with the effectiveness of the biological activity of the active ingredient(s). The characteristics of the carrier will depend on the route of administration. The pharmaceutical composition of the invention may also contain other anti-angiogenic and anti-tumor agents such cytokines or chemotherapeutic agents.

In practicing use of an antibody as provided herein, a therapeutically effective amount of antibody is administered to a human having a condition to be treated. The antibody may be administered in accordance with the methods herein either alone or in combination with other therapies such as treatments employing other hematopoietic factors (*e.g*., cytokines), chemotherapeutic agents, anti-angiogenic agents, and the like. When co-administered with one or more biologically active agents, the antibody provided herein may be administered either simultaneously with the biologically active agent(s), or sequentially. If administered sequentially, the attending physician will decide on the appropriate sequence of administering protein of the present invention in combination with the biologically active agent(s). Toxicity and therapeutic efficacy of such compounds can be determined by standard pharmaceutical procedures in cell cultures or experimental animals, *e.g*., for determining the LD₅₀ (the dose lethal to 50% of the population) and the ED₅₀ (the dose therapeutically effective in 50% of the population). The dose ratio between toxic and therapeutic effects is the therapeutic index and it can be expressed as the ratio between LD₅₀ and ED₅₀. Antibodies exhibiting high therapeutic indices are preferred. The data obtained from these cell culture assays and animal studies can be used in formulating a range of dosage for use in human. The dosage of such compounds lies preferably within a range of circulating concentrations that include the ED₅₀ with little or no toxicity. The dosage may vary within this range depending upon the dosage form employed and the route of administration utilized. The exact formulation, route of administration and dosage can be chosen by the individual physician in view of the patient's condition. *See, e.g.,* Fingl et al., THE PHARMACOLOGICAL BASIS OF THERAPEUTICS 1 (1975). Dosage amount and interval may be adjusted individually to provide plasma levels of the active moiety sufficient to maintain the desired therapeutic effects, or minimal effective concentration (MEC). The MEC will vary for each compound but can be estimated from *in vitro* data; for example, the concentration necessary to achieve 50-90% inhibition of migration activity using the assays described herein.

The mode of administration is not particularly important. In one embodiment, the mode of administration is an I.V. bolus. The prescribing physician will normally determine the dosage of the antibodies provided herein. It is to be expected that the dosage will vary according to the age, weight and response of the individual patient.

Techniques for formulation and administration of the antibodies for use in accordance with the invention may be found in REMINGTON'S PHARMACEUTICAL SCIENCES, Mack Publishing Co., Easton, Pa., latest edition. Suitable routes of administration may, for example, include oral, rectal, transmucosal, or intestinal administration; parenteral delivery, including intramuscular, subcutaneous, intramedullary injections, as well as intrathecal, direct intraventricular, intravenous, intraperitoneal, intranasal, or intraocular injections. Administration of antibody used in the pharmaceutical composition or to practice the method of the present invention can be carried out in a variety of conventional ways, such as oral ingestion, inhalation, topical application or cutaneous, subcutaneous, intraperitoneal, parenteral, intraarterial or intravenous injection. Intravenous administration to the patient is preferred.

Alternately, one may administer the antibody in a local rather than systemic manner, for example, via injection of the antibody directly into an arthritic joint, fibrotic tissue, or a tumor, often in a depot or sustained release formulation. Furthermore, one may administer the antibody in a targeted drug delivery system, for example, in a liposome coated with a tissue-specific antibody, targeting, for example, arthritic or fibrotic tissue or a tumor. The liposomes will be targeted to and taken up selectively by the afflicted tissue.

Pharmaceutical compositions for use in accordance with the invention thus may be formulated in a conventional manner using one or more physiologically acceptable carriers comprising excipients and auxiliaries that facilitate processing of the active compounds into preparations that can be used pharmaceutically. These pharmaceutical compositions may be manufactured in a manner that is itself known, *e.g*., by means of conventional mixing, dissolving, granulating, dragee-making, levigating, emulsifying, encapsulating, entrapping or lyophilizing processes. Proper formulation is dependent upon the route of administration chosen. When administered in liquid form, a liquid carrier such as water, petroleum, oils of animal or plant origin such as peanut oil, mineral oil, soybean oil, or sesame oil, or synthetic oils may be added. The liquid form of the pharmaceutical composition may further contain physiological saline solution, dextrose or other saccharide solution, or glycols such as ethylene glycol, propylene glycol or polyethylene glycol. When administered in liquid form, the pharmaceutical composition contains from about 0.5 to 90% by weight of protein of the present invention, and preferably from about 1 to 50% protein of the present invention.

When a therapeutically effective amount of antibody in accordance with the invention is administered by intravenous, cutaneous or subcutaneous injection, protein of the present invention will be in the form of a pyrogen-free, parenterally acceptable aqueous solution. The preparation of such parenterally acceptable protein solutions, having due regard to pH, isotonicity, stability, and the like, is within the skill in the art. A preferred pharmaceutical composition for intravenous, cutaneous, or subcutaneous injection should contain, in addition to protein of the present invention, an isotonic vehicle such as Sodium Chloride Injection, Ringer's Injection, Dextrose Injection, Dextrose and Sodium Chloride Injection, Lactated Ringer's Injection, or other vehicle as known in the art. The pharmaceutical composition of the present invention may also contain stabilizers, preservatives, buffers, antioxidants, or other additives known to those of skill in the art. For injection, the agents of the invention may be formulated in aqueous solutions, preferably in physiologically compatible buffers such as Hanks' solution, Ringer's solution, or physiological saline buffer. For transmucosal administration, penetrants appropriate to the barrier to be permeated are used in the formulation. Such penetrants are generally known in the art.

For administration by inhalation, the antibodies for use according to the invention are conveniently delivered in the form of an aerosol spray presentation from pressurized packs or a nebuliser, with the use of a suitable propellant, *e.g*., dichlorodifluoromethane, trichlorofluoromethane, dichlorotetrafluoroethane, carbon dioxide or other suitable gas. In the case of a pressurized aerosol the dosage unit may be determined by providing a valve to deliver a metered amount. Capsules and cartridges of, *e.g*., gelatin for use in an inhaler or insufflator may be formulated containing a powder mix of the compound and a suitable powder base such as lactose or starch. The compounds may be formulated for parenteral administration by injection, *e.g*., by bolus injection or continuous infusion. Formulations for injection may be presented in unit dosage form, *e.g*., in ampules or in multi-dose containers, with an added preservative. The compositions may take such forms as suspensions, solutions or emulsions in oily or aqueous vehicles, and may contain formulatory agents such as suspending, stabilizing and/or dispersing agents.

Pharmaceutical formulations for parenteral administration include aqueous solutions of the active compounds in water-soluble form. Additionally, suspensions of the active compounds may be prepared as appropriate oily injection suspensions. Suitable lipophilic solvents or vehicles include fatty oils such as sesame oil, or synthetic fatty acid esters, such as ethyl oleate or triglycerides, or liposomes. Aqueous injection suspensions may contain substances that increase the viscosity of the suspension, such as sodium carboxymethyl cellulose, sorbitol, or dextran. Optionally, the suspension may also contain suitable stabilizers or agents that increase the solubility of the compounds to allow for the preparation of highly concentrated solutions. Alternatively, the active ingredient may be in powder form for constitution with a suitable vehicle, *e.g*., sterile pyrogen-free water, before use.

The amount of antibody to be used in accordance with the invention in a pharmaceutical composition will depend upon the nature and severity of the condition being treated, and on the nature of prior treatments that the patient has undergone. Ultimately, the attending physician will decide the amount of protein of the present invention with which to treat each individual patient. Initially, the attending physician will administer low doses of antibodies of the present methods and observe the patient's response. Larger doses of antibodies of the present invention may be administered until the optimal therapeutic effect is obtained for the patient, and at that point the dosage is not increased further. It is contemplated that the various pharmaceutical compositions used to practice the methods herein should contain about 0.01 µg to about 100 mg (preferably about 0.1 µg to about 10 mg, more preferably about 0.1 µg to about 1 mg) of antibody of the present invention per kg body weight. When administered, the therapeutic composition for use in this invention is, of course, in a pyrogen-free, physiologically acceptable form. Therapeutically useful agents other than the required antibody may also optionally be included in the composition as described above; they may alternatively or additionally, be administered simultaneously or sequentially with such a composition.

The antibody specific for TEM 1 can be administered alone or in combination with other therapeutic modalities. For example, the treatment method can further comprise a step of delivering ionizing radiation to the cells contacted with the antibody. The ionizing radiation is delivered in a dose sufficient to induce a substantial degree of cell killing among the malignantly proliferating cells, as judged by assays measuring viable malignant cells. The degree of cell killing induced is substantially greater than that induced by either the antibody alone or the ionizing radiation alone. Typical forms of ionizing radiation include beta rays, gamma rays, alpha particles, and X-rays. These can be delivered from an outside source, such as X-ray machine or a gamma camera, or delivered to the malignant tissue from radionuclides administered to the patient. The use of radionuclides is well understood in the art and need not be detailed further. The use of ionizing radiation in the treatment of malignancies is described, for example, in S. Hellman, Principles of Radiation Therapy, in CANCER: PRINCIPLES & PRACTICE OF ONCOLOGY 248 (V. T. DeVita, Jr., et al., eds., 4th ed., 1993). A range of dosages that can be used is between about 1 and 500 cGy (*i.e*., from about 1 to about 500 rads).

The antibody can be administered alone or in combination with other antibodies identified as inhibitors of endothelial cell proliferation, migration, and/or tubule formation using the methods disclosed herein. The co-administered antibodies can be specific for different epitopes of the same TEM, different TEM, or a TEM and another angiogenesis-inhibitory or antitumor target.

Any cancerous disease where angiogenesis is implicated can be treated in accordance with the invention. Such diseases include, but are not limited to neoplasma of the central nervous system: glioblastomamultiforme, astrocytoma, oligodendroglial tumors, ependymal and choroids plexus tumors, pineal tumors, neuronal tumors, medulloblastoma, schwannoma, meningioma, meningeal sarcoma: neoplasma of the eye: basal cell carcinoma, squamous cell carcinoma, melanoma, rhabdomyosarcoma, retinoblastoma; neoplasma of the enbdocrine glands: pituitary neoplasms, neoplasms of the thyroid, neoplasms of the adrenal cortex, neoplasms of the neuroendocrine system, neoplasms of the gastroenteropancreatic endocrine system, neoplasms of the gonads; neoplasms of the head and neck: head and neck cancer, oral cavity, pharynx, larynx, odontogenic tumors: neoplasms of the thorax: large cell lung carcinoma, small cell lung carcinoma, non-small cell lung carcinoma, neoplasms of the thorax, malignant mesothelioma, thymomas, primary germ cell tumors of the thorax; neoplasms of the alimentary canal: neoplasms of the esophagus, neoplasms of the stomach, neoplasms of the liver, neoplasms of the gallbladder, neoplasms of the exocrine pancreas, neoplasms of the small intestine, vermiform appendix and peritoneum, adenocarcinoma of the colon and rectum, neoplasms of the anus; neoplasms of the genitourinary tract: renal cell carcinoma, neoplasms of the renal pelvis and ureter, neoplasms of the bladder, neoplasms of the urethra, neoplasms of the prostate, neoplasms of the penis, neoplasms of the testis; neoplasms of the female reproductive organs: neoplasms of the vulva and vagina, neoplasms of the cervix, adenocarcinoma of the uterine corpus, ovarian cancer, gynecologic sarcomas; neoplasms of the breast; neoplasms of the skin: basal cell carcinoma, squamous carcinoma, dermatofibrosarcoma, Merkel cell tumor; malignant melanoma; neoplasms of the bone and soft tissue: osteogenic sarcoma, malignant fibrous histiocytoma, chrondrosarcoma, Ewing's sarcoma, primitive neuroectodermal tumor, angiosarcoma; neoplasms of the hematipoeitic system: myelodysplastic syndromes, acute myeloid leukemia, chronic myeloid leukemia, acute lymphocytic leukemia, HTLV-1, and T-cell leukemia/lymphoma, chronic lymphocytic leukemia, hairy cell leukemia, Hodgkin's disease, non-Hodgkin's lymphomas, mast cell leukemia; neoplasms of children: acute lymphoblastic leukemia, acute myelocytic leukemias, neuroblastoma, bone tumors, rhabdomyosarcoma, lymphomas, renal and liver tumors.

The therapeutic antibody uses provided herein also provide foundation for important non-clinical uses as in bioassays of angiostasis and angiogenesis. By providing both positive and negative controls in assays of angiogenesis, such methods may be used to examine and characterize the effectiveness of candidate anti-angiogenic molecules while assessing potential toxic effects on normal angiogenesis.

Anti-angiogenic molecules are those molecules that reduce or eliminate angiogenesis. Such inhibition can occur through direct binding of one or more critical binding residues of a TEM protein that results in an anti-angiogenic signal, steric hindrance, reduced cell surface expression, and the like. As used herein, the term "anti-angiogenic molecule" includes both protein and non-protein moieties. In one embodiment, the agent is a small molecule. In another embodiment, the agent is a protein.

Disclosed herein, but not part of the claimed invention, is a method for identifying an angiogenesis inhibitor molecule that modulates a TEM protein, which comprises contacting a TEM-transgenic mouse bearing a tumor with a test molecule; and detecting the inhibition of the tumor growth, whereby the test molecule is identified as an angiogenesis inhibitor molecule that modulates the TEM protein or activity when the tumor growth in the mouse contacted with the test molecule is reduced relative to the tumor growth in the mouse not contacted by the test molecule. In one embodiment, the TEM is TEM 1. In another embodiment, the TEM is TEM 9. In yet another embodiment, the TEM is TEM 17.

The TEM transgenic mouse is generated using conventional transgenic methods. In one embodiment, the human TEM 1 cDNA in the RPCI11-867G23 BAC clone (GenBank Accession No. AP001107 Invitrogen Corp.) as a transgene. Injection into embryos from C57B1/6 mice was performed by YS New Technology Institute, Inc. The resulting TEM 1 transgenic mice are then crossbred with C57B1/6 mice (Japan SLC, Inc.) to produce additional transgenic progeny.

Any suitable tumor can be injected in any suitable manner to provide a model for the testing of anti-angiogenic molecules. The murine recipient of the tumor can be any suitable strain. The tumor can be syngeneic, allogeneic, or xenogenic to the tumor. The recipient can be immunocompetent or immunocompromised in one or more immune-related functions, included but not limited to nu/nu, scid, and beige mice. In one embodiment, the recipient is a transgenic mouse. In one specific embodiment, the transgenic mouse is a C57B1/6 mouse with a human TEM 1 transgene. The human TEM 1 transgene comprises the DNA sequence of TEM 1 in the RPCI11-867G23 BAC clone (GenBank Accession No. AP001107, Invitrogen Corp.). The TEM 1 transgene is then injected into C57BL/6 mice embryos using conventional methods in the art. The TEM 1 transgenic mouse can then be crossbred with non-transgenic, strain identical mice to expand the number of transgenic progeny.
The TEM 1 transgenic mice can be inoculated with syngeneic tumor cells, *e.g*., B16 melanoma (ATCC). The effect of antibody administration on tumor growth can be ascertained by quantifying the primary or metastatic tumor growth using conventional methods. The dosage of antibody ranges from 1µg/mouse to 1mg/mouse in at least one administration. The antibody can be administered by any suitable route. In one embodiment, the dose of antibody is 100 µg/mouse twice a week. In one specific embodiment, the B16 melanoma tumor is injected subcutaneously at day 0 into C57B1/6 human TEM 1 transgenic mice, and the volume of the primary tumor is measured at designated time points by using calipers. Any suitable control antibody can be used. In one example, the control antibody is a purified IgG1 isotype control antibody which had been raised against a hapten, dinitrophenyl.

A variety of different test anti-angiogenic molecules may be identified. Anti-angiogenic molecules can encompass numerous chemical classes. In certain embodiments, they are organic molecules, preferably small organic compounds having a molecular weight of more than 50 and less than about 2,500 daltons. Anti-angiogenic molecules can comprise functional groups necessary for structural interaction with proteins, particularly hydrogen bonding, and may include at least an amine, carbonyl, hydroxyl or carboxyl group, preferably at least two of the functional chemical groups. The anti-angiogenic molecules can comprise cyclical carbon or heterocyclic structures and/or aromatic or polyaromatic structures substituted with one or more of the above functional groups. Anti-angiogenic molecules also include biomolecules like peptides, saccharides, fatty acids, steroids, purines, pyrimidines, derivatives, structural analogs or combinations thereof. Test anti-angiogenic molecules of interest also can include peptide and protein agents, such as antibodies or binding fragments or mimetics thereof, *e.g*., Fv, F(ab')₂ and Fab.

Test anti-angiogenic molecules also can be obtained from a wide variety of sources including libraries of synthetic or natural compounds. For example, numerous means are available for random and directed synthesis of a wide variety of organic compounds and biomolecules, including expression of randomized oligonucleotides and oligopeptides. Alternatively, libraries of natural compounds in the form of bacterial, fungal, plant and animal extracts are available or readily produced. Additionally, natural or synthetically produced libraries and compounds are readily modified through conventional chemical, physical and biochemical means, and may be used to produce combinatorial libraries. Known pharmacological agents may be subjected to directed or random chemical modifications, such as acylation, alkylation, esterification, amidification, etc. to produce structural analogs.

A test anti-angiogenic molecule is identified as an inhibitor when it is capable of specifically inhibiting the growth of a tumor by at least 20%, often 30, 40, 50, 60, 70, 80 or 90%, and sometimes 100%. The growth inhibition can be quantified using any convenient method of measurement. For example, for primary tumor growth, perpendicular measurements are taken of the tumor mass to calculate tumor volume. Metastatic growth can be ascertained by microscopic or macroscopic analysis, as appropriate. The tumor can be syngeneic, allogeneic, or xenogeneic to the transgenic animal. The test molecules can be administered at the time of tumor inoculation, after the establishment of primary tumor growth, or after the establishment of local and/or distant metastases. Single or multiple administration of the test molecule can be given using any convenient mode of administration, including but not limited to intravenous, intraperitoneal, intratumoral, subcutaneous, and intradermal.

The following examples illustrate the claimed invention in so far as they relate to TEM 1 antibodies, including their generation and testing.

### Examples

### Example 1

### Endothelial Precursor Cells (EPC's)

Bone marrow cells expressing the endothelial cell lineage markers AC133 and CD34 can be stimulated with VEGF, bFGF and heparin in fibronectin or collagen monolayer culture to differentiate into a phenotype described as endothelial precursor cells (EPCs). Briefly, todifferentiate bone marrow cells into EPC's, the AC133⁺/CD34⁺ bone marrow population is isolated using conventional means. In suspension culture, the AC133⁺/CD34⁺ cells are stimulated with 50 ng/ml VEGF₁₆₅, 10-50 ng/ml bFGF, and 50/ml heparin in IMDM media supplemental with 15% FBS. The cells undergo rapid proliferation and differentiation. The resulting adherent cell population is then employed in the *in vitro* assays examining TEM function. These EPCs can be expanded in culture for over a dozen passages and appear to be an intermediary between stem cells from bone marrow and fully differentiated endothelial cells. Characterization of these EPCs indicate that they possess many of the same properties as mature endothelial cells such as HMVECs and HUVECs. *In vitro,* EPCs can form tubes on Matrigel, migrate and invade, important properties in the formation of new vessels within a tumor microenvironment. SAGE analysis of EPCs has been performed comparing gene expression under stimulatory (+ VEGF) and non-stimulatory (- VEGF) conditions with notable differences being observed. *In vivo* human EPCs can form tubes when implanted in Matrigel plugs in immunodeficient mice thus allowing model of angiogenesis with human endothelium in mice. EPCs have been found to express many endothelial cell surface markers including CD31, P1H12, and CD105 at levels similar to those of HUVEC and HMVEC as analyzed by flow cytometry. In addition, EPCs express colon tumor endothelial markers (TEMs), mRNAs that have been identified as being expressed at high levels in endothelial cells derived from a fresh surgical specimen of colon tumor compared to the endothelium of normal colon mucosa. The levels of TEM mRNA expressed in human EPCs were markedly higher than was observed in MHVECs and HUVECs. In fact, many TEMs were not detectable in HUVECs and MHVECs.

### Example 2

### TEM 1 Antibody

To generate a murine polyclonal antibody to TEM 1, C57B1/6 mice were immunized intramuscularly with pcDNA 3.1 human TEM 1 (hTEM 1) plasmids containing the full-length human gene for TEM 1. Human TEM1 coding sequence was cloned from human fetal brain RNA by RT-PCR using 3' and 5' TEM1 specific primers. The 5' end primer was modified to contain a consensus Kozak sequence of CCACC 5' to the ATG start codon. The amplified product, a 2273 basepair (bp) fragment corresponding to nucleotide (nt) 6 - nt 2279 of SEQ ID NO. 195 of U.S. Serial No. 09/918715 (Publication No. 20030017157) (GenBank # NM_020404), was sequence verified and subcloned into pCDNA3.1 vector (Invitrogen). This vector pcDNA3.1 hTEM1 encodes the full length protein of 757 amino acids.

To generate a polyclonal specific for the TEM 1 extracellular domain, nt102 to nt1963 of the full-length human gene for TEM 1 was subcloned into VV-1 vector (Genovac AG, Freiburg, Germany) inframe, with the 5' signal sequence and myc-TAG at the 5' end and a GPI transmembrane anchor motif at the 3' end in the vector, to generate VV-1 TEM1. The extracellular portion included in the vector encodes TEM1 from amino acids 33 to 684 of SEQ ID NO. 196 of U.S. Serial No. 09/918715 (Publication No. 20030017157) (GenBank # NM_020404). Rabbits were immunized with the VV1-TEM 1 vector. The IgG fractions for the rabbit polyclonals were then purified using conventional methods.

Cell Surface Expression: To determine if TEM 1 is found on the surface of cells, COS cells (ATCC) were transiently transfected with the plasmid vector, pCFA-TEM1. The full- length human TEM 1 was cloned into the pCFA vector backbone (Yew et al., Hum. Gene Ther. 10: 223-34 (1999). The cells (non-permeablized) were then stained with the rabbit anti-TEM 1 polyclonal, followed by staining with the CY3-labeled anti-rabbit Ig antibody. Cells were then counterstained with a nuclear stain (DAPI) and examined by fluorescent light microscopy. The fluorescent staining was confined to the cell surface of the COS cells, thus demonstrating that TEM 1 is expressed on the surface of the transfected cells.

Using flow cytometric analysis, the cell surface expression of TEM 1 was confirmed using the murine polyclonal anti human TEM 1 antibody on 2H1 endothelial cells (2H11 cells endogenously express murine TEM 1) and using Hek-293 cells transfected with VV1-TEM 1 and treated with the rabbit polyclonal anti human TEM 1. Both cells stained positively with the anti-TEM 1 antibody. These experiments prove, for the first time, that TEM 1 is an extracellular and membrane localized protein.

Proliferation Assay: Proliferation was assayed by the methods described in Crouch et al., J. Immunol. Meth. 160: 81 (1993) and Kangas et al., Med. Biol. 62: 338 (1984) using human endothelial precursor cells (EPC's) prepared as described in Example 1. Proliferation was assessed in a 96 well plate system using 2x10³ cell/well in media supplemented with 2% fetal bovine serum. The ATP luminescence assay (CellTiter Glo™ Luminescent Cell Viability kit, Promega) was used as a growth inhibition endpoint for human EPC's exposed to rabbit polyclonal anti-human TEM 1. Cells were incubated with increasing concentrations of antibody from 100 - 800 µg/ml for 48-hours. The control IgG antibody was rabbit serum IgG fraction purchased from Sigma. In the absence of antibody, 3,662 ± 354 EPC's were detected. At 200, 400 and 800 µg/ml of antibody, the cell numbers observed in the presence of control IgG were 4,185 ± 117, 4,418 ± 38, and 4,611 ± 165, respectively. In contrast, the cell numbers observed in the presence of anti-TEM 1 antibody at 200, 400, and 800 µg/ml were 3,756 ± 92, 3,881 ± 97, and 3,773 ± 127, respectively, demonstrating the anti-proliferative effect of anti-TEM 1 antibody. This anti-proliferative effect was also observed in murine 2H11 endothelial cells treated with rabbit anti-TEM 1 polyclonal antibody. In the absence of antibody, 2H11 cells produced 9,216 ± 102 cells. 2H11 cells incubated with 200, 400, or 800 µg/ml of control IgG produced 10,672 ± 292 cells, 10,846 ± 475 cells, and 11,977 ± 267 cells, respectively. In the presence of the anti-TEM 1 antibody, however, the cell numbers were reduced to 10,205 ± 113 cells, 10,269 ± 114 cells, and 8,725 ± 97 cells, in the presence of 200, 400, and 800 µg/ml, respectively.

Migration Assay: Migration was assayed as described in Glaser et al., Nature 288: 483-84 (1983) and Alessandri et al., Cancer Res. 43: 1790-97 (1983) using human EPC's (prepared as described in Example 1) plated in media without serum in the upper chamber atop the 8 micron pore membrane at 5x10⁴ per chamber. Media supplemented with 0.5% fetal bovine serum was placed as a chemo-attractant in the lower chamber. The cells were allowed to migrate through the lower side of the membrane over a 48-hour period in the presence and absence of 800 µg/ml anti-human TEM 1 rabbit polyclonal antiserum or control rabbit IgG fraction. The antibody was placed in both the upper and lower chambers of the experimental wells. The cells that migrated through the membrane were then stained with calcein and quantified by fluorescence intensity. After 48 hours, migrating cells produced 7000 and 7750 RFUs, respectively, when incubated without antibody or in the presence of control IgG. However, in the presence of anti-TEM 1 antibody, the RFUs were reduced to 4200, indicating a significant reduction in the number of migrating cells.

Endothelial Tube Formation Assay: An adenovirus vector containing the gene encoding the full length human TEM 1 (Ad2CMV-TEM1) was constructed using the pAD(vantage) system as described in Souza, et al., Biotechniques, 26:502-08(1999). Ad2CMV-TEM1 was used to infect human microvascular endothelial cells (HMVEC) at an MOI of 300. Seventy-two hours post-viral infection, the HMVEC were trypsinized and plated onto Matrigel™ for the tube formation assay. The assay was carried out in a 24 well plate for 24 hours. Cells were then stained with Calcein AM (Molecular Probes) for 30-60 minutes at 37°C, and fluorescence images of the tubes were captured. The area occupied by the tubes was quantified using MetaMorph image analysis. Cells were plated at a density of 3x10⁴ cells/well with 250 µl of Matrigel™. In the absence of antibody, the tube area for the non-infected, empty vector (EV)-infected, and TEM 1-infected cells was 90000, 61000, and 110000 pixels, respectively. In the presence of control antibody, the tube area for non-infected, empty vector (EV)-infected, and TEM 1-infected cells was 38000, 62000, and 61000 pixels, respectively. In the presence of 800 µg/ml anti-TEM 1 rabbit polyclonal antiserum, a decrease in tube formation was observed in the adeno-TEM 1-infected HMVEC relative to the cells exposed to pre-immune rabbit serum. The tube area for the non-infected, empty vector (EV)-infected, and TEM 1-infected cells was 38000, 80000, and 42000, respectively. The anti-TEM 1 antibody treated cells had a 63% decrease in tube area relative to tube area in the cells not exposed to anti-TEM 1 antibody.

### Example 3

### TEM 17 Antibody

To generate a polyclonal specific for the TEM 17 extracellular domain, nt 152 to nt 1318 of the full length human gene for TEM17, was subcloned into VV-1 vector (Genovac AG, Freiburg, Germany) inframe with the 5' signal sequence and myc-tag at the 5'end and a GPI transmembrane anchor motif at the 3' end in the vector to generate VV-1 TEM 17. The extracellular portion included in the vector encodes TEM17 from amino acids 24 to 412 of SEQ ID NO. 230 of U.S. Serial No. 09/918715 (Publication No. 20030017157). Rabbits were immunized with the VV1-TEM 17 vector.

To generate a murine polyclonal antibody to TEM 17, C57B1/6 mice were immunized intramuscularly with pcDNA 3.1 hTEM 17 plasmids containing the full-length human gene for TEM 17. Human TEM 17 coding sequence was cloned from human fetal brain RNA by RT-PCR using 3' and 5' TEM17 specific primers. The 5'end primer was modified to contain a consensus Kozak sequence of CCACC 5' to the ATG start codon. The amplified product of 1503 bp which corresponds to nt 83 - nt 1585 of SEQ. ID NO. 229 of U.S. Serial No. 09/918715 (Publication No. 20030017157) (GenBank # AF279144) was sequence verified and subcloned into pCDNA3.1 vector (Invitrogen). Thus, pcDNA3.1 hTEM17 encodes the full length protein of 500 amino acids. The IgG fractions for the rabbit and murine polyclonals were then purified using conventional methods.

Cell Surface Expression: To determine if TEM 17 is found on the surface of cells, COS cells (ATCC) were transiently transfected with the plasmid vector, pCFA-TEM17. The full- length human TEM 17 was cloned into the pCFA vector backbone. The pCFA vector backbone is described in Yew et al., Hum. Gene Ther. 10: 223-34 (1999). The cells (non-permeablized) were then stained with the rabbit anti-TEM 17 polyclonal, followed by staining with the CY3-labeled anti-rabbit Ig antibody. Cells were then counterstained with a nuclear stain (DAPI) and examined by fluorescent light microscopy. The fluorescent staining was confined to the cell surface of the COS cells, thus demonstrating that TEM 17 is expressed on the surface of the transfected cells.

Using flow cytometric analysis, the cell surface expression of TEM 17 was confirmed using the murine polyclonal anti human TEM 17 antibody on 2H11 endothelial cells (2H11 cells endogenously express murine TEM 17) and using Hek-293 cells transfected with VV1-TEM 17 and treated with the rabbit polyclonal anti human TEM 17. Both cell types stained positive using the anti-TEM 17 antibodies. These experiments prove, for the first time, that TEM 17 is an extracellular and membrane localized protein.

Proliferation Assay: Proliferation was assayed by the methods described in Crouch et al., J. Immunol. Meth. 160: 81 (1993) and Kangas et al., Med. Biol. 62: 338 (1984) using human endothelial precursor cells (EPC's) prepared as described in Example 1. Proliferation was assessed in a 96 well plate system using 2x10³ cell/well in media supplemented with 2% fetal bovine serum. The ATP luminescence assay (CellTiter Glo™ Luminescent Cell Viability kit, Promega) was used as a growth inhibition endpoint for human EPC's exposed to rabbit polyclonal anti-human TEM 17. Cells were incubated with increasing concentrations of antibody from 100 - 800 µg/ml for 48-hours. The control IgG antibody was rabbit serum IgG fraction purchased from Sigma. In the absence of antibody, 3,662 ± 354 EPC's were detected. At 200, 400 and 800 µg/ml of antibody, the cell numbers observed in the presence of control IgG were 4,185 + 117, 4,418 ± 38, and 4,611 ± 165, respectively. In contrast, the cell numbers observed in the presence of anti-TEM 17 antibody at 200, 400, and 800 µg/ml were 3,480 ± 190, 3,472 ± 35, and 3,536 ± 166, respectively, demonstrating the anti-proliferative effect of anti-TEM 17 antibody. This anti-proliferative effect was not observed in murine 2H11 endothelial cells treated with rabbit anti-TEM 17 polyclonal antibody.

Migration Assay: Migration was assayed as described in Glaser et al., Nature 288: 483-84 (1983) and Alessandri et al., Cancer Res. 43: 1790-97 (1983) using human EPC's (prepared as described in Example 1) plated in media without serum in the upper chamber atop the 8 micron pore membrane at 5x10⁴ per chamber. Media supplemented with 0.5% fetal bovine serum was placed as a chemo-attractant in the lower chamber. The cells were allowed to migrate through the lower side of the membrane over a 48-hour period in the presence and absence of 800 µg/ml anti-human TEM 17 rabbit polyclonal antiserum or control rabbit IgG fraction. The antibody was placed in both the upper and lower chambers of the experimental wells. The cells that migrated through the membrane were then stained with calcein and quantified by fluorescence intensity. After 48 hours, migrating cells produced 4500 and 10,000 RFUs, respectively, when incubated without antibody or in the presence of control IgG. However, in the presence of anti-TEM 17 antibody, the RFUs were reduced to 3500, indicating a significant reduction in the number of migrating cells relative to the control antibody treated cells.

Endothelial Tube Formation Assay: To examine the effect of anti-TEM 17 antibody on endothelial cell tube formation, human EPCs (prepared as described in Example 1) were pre-incubated overnight in the presence of rabbit anti-human TEM 17 polyclonal antibody, and then were plated in basal media supplemented with 0.5% and 2% fetal bovine serum (FBS) at 2x10⁴ cell/well in a 48 well dish pre-coated with Matrigel™. After 24 hours, cells were stained with Calcein AM (Molecular Probes) for 30-60 minutes at 37°C, and fluorescence images of the tubes were captured. The area occupied by the tubes was quantified using MetaMorph image analysis. In the presence of control antibody, the tube area for EPCs was 3000 and 2800 pixels, respectively, in the presence of 0.5% and 2% FBS, respectively. In the presence of 800 µg/ml anti-TEM 17 rabbit polyclonal antiserum, the tube area for EPCs was 2200 and 600 pixels, respectively, in the presence of 0.5% and 2% FBS, respectively, demonstrating the potent inhibitory effect of the anti-TEM 17 antibody. Similarly, the tube formation by 2H11 endothelial cells were inhibited in the presence of rabbit polyclonal anti-TEM 17. 2H11 cells were pre-incubated with 800 mg/ml of antibody, and then allowed to form networks/tubes in Matrigel for five hours. In the presence of control IgG, the tube area was 6000 pixels whereas in the presence of anti-TEM 17 antibody, the area was 2300 pixels, indicating that anti-TEM 17 antibody provides an inhibitory effect on tubule formation of both human EPCs and 2H1 endothelial cells.

### Example 4

### TEM 9 Antibody

To generate a murine polyclonal antibody to TEM 9, C57B1/6 mice were immunized intramuscularly with pcDNA 3.1 hTEM9 plasmids containing the full-length human gene for TEM 9. Human TEM9 cDNA was cloned by colony hybridization of human Fetal Kidney cDNA library in pCMV Sport2. cDNA was screened using a 1100bp PCR fragment encoding TEM9 sequence and positive clones were sequenced to completion. The cDNA library was created by adding Not1 and Sal1 sites at the 3' and 5'end of the cDNA. One of the clones pCMV Sport-2 TEM9 (Clone 9-1) that that contains the full length TEM9 ORF of 3996 bp identical to nt 35 to nt 4030 of SEQ ID NO. 211 of U.S. Serial No. 09/918715 (Publication No. 20030017157) (AF 378755) was selected. The full length sequence from pCMV Sport-2 TEM9 was subcloned into pCDNA3.1 vector (Invitrogen) to generate pcDNA3.1 hTEM9, which was used in the immunizations of the mice.

To generate a polyclonal specific for the TEM 9 extracellular domain, nt 127 to nt 2290 of the full length human gene for TEM 9, was subcloned into the VV-1 vector (Genovac AG, Freiburg, Germany) inframe with the 5' signal sequence and myc-tag at the 5'end and a GPI transmembrane anchor motif at the 3' end in the vector, to generate VV-1 TEM 9. The extracellular portion included in the vector encodes TEM9 from amino acids 32 to 752 of SEQ ID NO. 212 of U.S. Serial No. 09/918715 (Publication No. 20030017157). Rabbits were immunized with the VV1-TEM 9 vector. The IgG fractions for the rabbit and murine polyclonals were then purified using conventional methods.

Cell Surface Expression: To determine if TEM 9 is found on the surface of cells, COS cells (ATCC) were transiently transfected with the plasmid vector, pCFA-TEM9. The full- length human TEM 9 was cloned into the pCFA vector backbone. The pCFA vector backbone is described in Yew et al., Hum. Gene Ther. 10: 223-34 (1999). The cells (non-permeablized) were then stained with the rabbit anti-TEM 9 polyclonal, followed by staining with the CY3-labeled anti-rabbit Ig antibody. Cells were then counterstained with a nuclear stain (DAPI) and examined by fluorescent light microscopy. The fluorescent staining was confined to the cell surface of the COS cells, thus demonstrating that TEM 9 is expressed on the surface of the transfected cells.

Using flow cytometric analysis, the cell surface expression of TEM 9 was confirmed using the murine polyclonal anti human TEM 9 antibody on 2H11 endothelial cells (2H11 cells endogenously express murine TEM 9) and using Hek-293 cells transfected with VV1-TEM 9 and treated with the rabbit polyclonal anti human TEM 9. Both cell types stained positive with the anti-TEM 9 antibody. These experiments prove, for the first time, that TEM 9 is an extracellular and membrane localized protein.

Proliferation Assay: Proliferation was assayed by the methods described in Crouch et al., J. Immunol. Meth. 160: 81 (1993) and Kangas et al., Med. Biol. 62: 338 (1984) using human endothelial precursor cells (EPC's) prepared as described in Example 1. Proliferation was assessed in a 96 well plate system using 2x10³ cell/well in media supplemented with 2% fetal bovine serum. The ATP luminescence assay (CellTiter Glo™ Luminescent Cell Viability kit, Promega) was used as a growth inhibition endpoint for human EPC's exposed to rabbit polyclonal anti-human TEM 9. Cells were incubated with increasing concentrations of antibody from 100 - 800 µg/ml for 48-hours. The control IgG antibody was rabbit serum IgG fraction purchased from Sigma. No inhibition of proliferation was observed for human EPCs or murine 2H11 cells.

Migration Assay: Migration was assayed as described in Glaser et al., Nature 288: 483-84 (1983) and Alessandri et al., Cancer Res. 43: 1790-97 (1983) using human EPC's (prepared as described in Example 1) plated in media without serum in the upper chamber atop the 8 micron pore membrane at 5x10⁴ per chamber. The 2H11 cells were pre-incubated with antibody for 30 minutes prior to plating. Media supplemented with 0.5% fetal bovine serum was placed as a chemo-attractant in the lower chamber. The EPCs and 2H11 cells were allowed to migrate through the lower side of the membrane over a 48- and 4-hour period, respectively, in the presence and absence of 800 µg/ml anti-human TEM 9 rabbit polyclonal antiserum or control rabbit IgG fraction. The antibody was placed in both the upper and lower chambers of the experimental wells. The cells that migrated through the membrane were then stained with calcein and quantified by fluorescence intensity. Incubation with control serum alone resulted in 1000 RFU produced by the migrating 2H11 cells while anti-TEM 9 antibody reduced the relative fluorescence to 820 RFUs, indicating inhibition of migration by the TEM antibody. A similar inhibition was observed with EPCs in the presence of anti-TEM 9 antibody. Inhibition of migration by anti-TEM 9 antibody was further confirmed using adeno-TEM 9 infected (Ad2CMV-TEM9) HMVECs prepared as described below. In the presence of control IgG, the migrating Adeno-TEM 9-infected EPCs had a fluorescent intensity of 1899. The presence of anti-TEM 9 antibody reduced the fluorescent intensity to 1254.

Endothelial Tube Formation Assay: An adenovirus vector containing the gene encoding the full length human TEM 9 (Ad2CMV-TEM9) was constructed using the pAD(vantage) system as described in Souza, et al., Biotechniques, 26:502-08(1999). Ad2CMV-TEM9 was used to infect human microvascular endothelial cells (HMVEC) at an MOI of 300. Seventy-two hours post-viral infection, the HMVEC were trypsinized and plated onto Matrigel™ for the tube formation assay. The assay was carried out in a 24 well plate for 24 hours. Cells were then stained with Calcein AM (Molecular Probes) for 30-60 minutes at 37°C, and fluorescence images of the tubes were captured. The area occupied by the tubes was quantified using MetaMorph image analysis. Cells were plated at a density of 3x10⁴ cells/well with 250 µl of Matrigel™. No decrease in tube area from the anti-TEM 9 treated cells as compared with the cells not exposed to anti-TEM 9 antibody

### Example 5

### Human anti-TEM 1 antibody

Human antibodies to TEM 1 were raised in the KM mouse™ (WO 02/043478) by immunizing TEM 1-expressing FM3A cells (FM3A/TEM1) or TEM 1-expressing L929 cells (L929/TEM 1), both of which stably express high levels of human TEM 1 as determined by flow cytometric (FACS) analysis using reagent rabbit polyclonal antibodies to human TEM 1. For the establishment of FM3A/TEM 1, murine FM3A mammary carcinoma cells (ATCC) were transfected with a plasmid containing the human TEM 1 cDNA by electroporation. The plasmid was constructed by subcloning TEM 1 cDNA from pcDNA3.1 human TEM 1 into pTracerEF-Bsd (Invitrogen) using BstXI site. For the establishment of L929/TEM1, murine L929 fibroblasts (ATCC) were transfected with the same plasmid by lipofection using TransIT-LT1 (Mirus). TEM 1-expressing cells were, then, collected from the transfectants stained with reagent rabbit polyclonal antibodies, using a cell sorter, FACSVantage (BD Biosciences). FM3A/TEM 1 was maintained in modified Eagle's medium supplemented with 10% Fetal Bovine Serum (FBS) and 10 µg/ml of Blasticidin S, and L929/TEM 1 was maintained in Dulbecco's Modified Eagle's Medium supplemented with 10% FBS and 10 µg/ml of Blasticidin S. The mice were immunized with 5x10⁶ cells of FM3A/TEM1 or 1x10⁶ cells of L929/TEM1 intraperitoneally 3 times with 2 weeks interval. At the last immunization, 3 days before cell fusion, 5 µg of human interleukin-6 was injected intraperitoneally into the immunized mice. Hybridomas were prepared from the spleens of the immunized animals using SP2/0-Ag14 myeloma cells (ATCC) as a fusion partner. Hybridomas which produce antibody against human TEM 1 were screened by FACS analysis of their supernatants using TEM 1-expressing FM3A cells. In brief, TEM 1-expressing FM3A cells were incubated with the supernatants at 0°C for one hour. After washing, the cells were incubated with RPE-conjugated anti-human kappa chain specific antibody or RPE-conjugated anti-human gamma chain specific antibody at 0°C for 1 hour. After washing, the cells were subjected to FACS analysis. Selected hybridomas were cloned by limiting dilution method.

Purification of Antibodies: For purification of antibodies, hybridomas were adapted to eRDF medium (Kyokuto Pharmacy) supplemented with 1% Low IgG FBS (HyClone), 5 µg/ml of insulin, 5 µg/ml of transferrin, 10 µM of ethanolamine, and 25 nM of sodium selenite. The purification of the IgG from the fermentation broth was performed using a combination of conventional techniques commonly used for antibody production. The culture harvest is clarified to remove cells and cellular debris prior to starting the purification scheme. This was achieved using filtration of the harvest. Following clarification, the antibody was captured and significantly purified using affinity chromatography on Protein A matrix (MabSelect; Amersham Biosciences). The antibody is bound to Protein A matrix and, following washing of the matrix, is eluted by a reduction of the pH. Further purification of the antibody is then achieved by anion exchange chromatography (Q Sepharose Fast Flow; Amersham Biosciences) and cation exchange chromatography (SP Sepharose Fast Flow; Amersham Biosciences). As well as removing impurities, this step can also be used to buffer exchange into PBS.

Proliferation Assay: Human endothelial precursor cell (EPC) proliferation was assessed in a 96 well plate system using 2x10³ cell/well in media supplemented with 2% fetal bovine serum. The ATP luminescence assay (CellTiter Glo™ Luminescent Cell Viability kit, Promega) was used as a growth inhibition endpoint for EPCs incubated with human antibodies. Cells were incubated with 1 µg/ml of human anti-TEM 1 supernatants for 48-hours. A number of antibody supernatants were inhibitory for EPC proliferation.

Inhibition of Tumor Growth by human TEM 1 Antibody: For *in vivo* evaluation of human TEM 1 antibodies against human TEM 1, human TEM 1 transgenic mice was established using RPCI11-867G23 BAC clone (Invitrogen Corporation) as a transgene. Injection of the transgene into C57B1/6 embryos was performed by YS New Technology Institute, Inc. The TEM 1 transgenic mice were then crossbred with non-transgenic C57BL/6 mice (Japan SLC, Inc.) to expand the transgenic progeny. Adult TEM 1 transgenic mice were inoculated 1x10⁶ cells of B16 melanoma (ATCC) subcutaneously at day 0, and tumor volume in individual mice from each experimental group was measured at designated time points using calipers. The purified TEM1 antibodies from clone 7, clone 38, and clone 70, and a purified IgG1 isotype control antibody specific for a hapten (*i.e.,* dinitrophenyl) were administered twice a week at a dose of 100 µg/mouse starting at day 1. At day 6, the tumor volume in the mice receiving the control IgG1 antibody (n=6), clone TEM1-7 antibody (n=6), clone TEM1-70 (n=4), and clone TEM1-38 (n=6) was 4.88 ± 3.629, 6.12 ± 6.12, 3.56 ± 3.56, 36.83 ± 15.15 mm³, respectively (values represent mean ± SEM). At day 9, the tumor volume in the mice receiving the IgG control antibody had a tumor volume of 379.02±112.90 mm³, while the mice receiving clone TEM1-7, clone TEM1-70, and clone TEM1-38 anti-TEM 1 antibodies were 221.82±54.12, 130.08±25.14, and 467.41±185.19 mm³, respectively. At day 13, the anti-TEM I antibody inhibited the B16 tumor growth even more dramatically. The tumor volume in the mice receiving only control antibody had increased to 944.04±402.19 mm³, while in the mice receiving the anti-TEM 1 antibodies the tumor volume was reduced to 204.88 ± 31.56, 128.67±62.46, and 513.92±174.40 mm³ for the clone TEM1-7, TEM1-70 and TEM1-38 antibodies, respectively.

## Claims

1. An unconjugated antibody, or an unconjugated biologically active fragment thereof, which specifically binds to human Tumor Endothelial Marker (TEM) 1 for use as medicament for inhibiting tumor growth.

2. An antibody for use according to claim 1 wherein the tumor is an adenocarcinoma, leukaemia, lymphoma, melanoma, sarcoma, orteratocarcinoma.

3. An antibody for use according to claim 1 wherein the tumor is a cancer of the adrenal gland, bladder, bone, bone marrow, brain, breast, cervix, gall bladder, ganglia, gastronintestinal tract, heart, kidney, liver, lung, muscle, ovary, pancreas, parathyroid, penis, prostate, salivary glands, skin, spleen, testis, thymus, thyroid, or uterus.

4. An antibody for use according to any of the preceding claims, wherein tumor growth is inhibited by inhibiting angiogenesis.

5. An antibody for use according to claim 4, wherein the angiogenesis is neoangiogenesis.

6. An antibody for use according to claim 4, wherein angiogenesis is inhibited by inhibiting endothelial tube formation.

## Patentansprüche

1. Unkonjugierter Antikörper oder ein unkonjugiertes, biologisch aktives Fragment dessen, der bzw. das speziell mit einem menschlichen Tumorendothelialmarker (TEM) 1 bindet, zur Verwendung als ein Arzneimittel zur Hemmung des Tumorwachstums.

2. Antikörper zur Verwendung nach Anspruch 1, wobei es sich bei dem Tumor um ein Adenokarzinom, Leukämie, ein Lymphom, ein Melanom, ein Sarkom oder ein Teratokarzinom handelt.

3. Antikörper zur Verwendung nach Anspruch 1, wobei es sich bei dem Tumor um ein Nebenkarzinom, ein Blasenkarzinom, ein Knochenkarzinom, ein Knochenmarkkarzinom, ein Gehirnkarzinom, ein Brustkarzinom, ein Gebärmutterhalskarzinom, ein Gallenblasenkatzinom, ein Ganglienkarzinom, ein Karzinom des Gastrointestinaltrakts, ein Herzkarzinom, ein Nierenkarzinom, ein Leberkarzinom, ein Lungenkarzinom, ein Ovarialkarzinom, ein Bauchspeicheldrüsenkarzinom, ein Nebenschilddrüsenkarzinom, ein Peniskarzinom, ein Prostatakarzinom, ein Speicheldrüsenkarzinom, ein Hautkarzinom, ein Milzkarzinom, ein Hodenkarzinom, ein Thymuskarzinom, ein Schilddrüsenkarzinom oder ein Gebärmutterkarzinom handelt.

4. Antikörper zur Verwendung nach einem der vorstehenden Ansprüche, wobei das Tumorwachstum durch hemmende Angiogenese gehemmt wird.

5. Antikörper zur Verwendung nach Anspruch 4, wobei es sich bei der Angiogenese um Neoangiogenese handelt.

6. Antikörper zur Verwendung nach Anspruch 4, wobei die Angiogenese gehemmt wird durch die Hemmung der Endothelial-Röhrenbildung.

## Revendications

1. Anticorps non conjugué, ou fragment biologiquement actif non conjugué de celui-ci, qui se fixe spécifiquement au marqueur endothélial tumoral (TEM) 1 humain destiné à être utilisé comme médicament pour inhiber la croissance tumorale.

2. Anticorps destiné à être utilisé selon la revendication 1, dans lequel la tumeur est un adénocarcinome, une leucémie, un lymphome, un mélanome, un sarcome ou un tératocarcinome.

3. Anticorps destiné à être utilisé selon la revendication 1, dans lequel la tumeur est un cancer de la glande surrénale, de la vessie, de l'os, de la moelle osseuse, du cerveau, du sein, du col de l'utérus, de la vésicule biliaire, du ganglion nerveux, du tractus gastrointestinal, du choeur, du rein, du foie, du poumon, du muscle, de l'ovaire, du pancréas, de la parathyroïde, du pénis, de la prostate, des glandes salivaires, de la peau, de la rate, du testicule, du thymus, de la thyroïde ou de l'utérus.

4. Anticorps destiné à être utilisé selon l'une quelconque des revendications précédentes, dans lequel la croissance tumorale est inhibée par l'inhibition de l'angiogenèse.

5. Anticorps destiné à être utilisé selon la revendication 4, dans lequel l'angiogenèse est la néoangiogenèse.

6. Anticorps destiné à être utilisé selon la revendication 4, dans lequel l'angiogenèse est inhibée par l'inhibition de la formation du tube endothélial.
